(19) Europäisches Patentamt
European Patent Office
Office européen des brevets

(11) Veröffentlichungsnummer: **0 124 752**
**B1**

(12) # EUROPÄISCHE PATENTSCHRIFT

(45) Veröffentlichungstag der Patentschrift: **16.07.86**

(51) Int. Cl.[4]: **A 23 K 1/16**

(21) Anmeldenummer: **84103520.7**

(22) Anmeldetag: **30.03.84**

(54) **Stoffmischungen mit wachstumsfördernder und leistungssteigernder Wirkung sowie Futtermittel und Tränkflüssigkeiten, die geringe Mengen dieser Stoffmischungen enthalten.**

(30) Priorität: **07.04.83 DE 3312425**

(43) Veröffentlichungstag der Anmeldung: **14.11.84 Patentblatt 84/46**

(45) Bekanntmachung des Hinweises auf die Patenterteilung: **16.07.86 Patentblatt 86/29**

(84) Benannte Vertragsstaaten: **BE CH DE FR GB LI NL**

(56) Entgegenhaltungen:
**EP - A - 0 065 192**
**EP - A - 0 083 008**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Lechtken, Peter, Dr., Ludwigshafener Strasse 6 b, D-6710 Frankenthal (DE)**
Erfinder: **Nuerrenbach, Axel, Dr., Koenigsberger Strasse 7, D-6718 Gruenstadt (DE)**
Erfinder: **Dr. Kohler Walter, Max-Slevogt-Strasse 2, D-6710 Frankenthal (DE)**
Erfinder: **Hoppe, Peter Paul, Dr., Am Hauenstein 13, D-6706 Wachenheim (DE)**
Erfinder: **Tiefenbacher, Hubert, Dr., Hauaeckerstrasse 49, D-7022 Leinfelden-Echterdingen (DE)**
Erfinder: **Schole, Juergen, Dr. Dipl.-Landwirt., Weissdornweg 19, D-3002 Wedemark 1 (DE)**

**EP 0 124 752 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft Stoffmischungen und diese enthaltende Futtermittel und Tränkflüssigkeiten mit wachstumsfördernder und/oder leistungssteigernder Wirkung, die bei Haus- und Nutztieren anzuwenden sind.

Bei der stetig wachsenden Weltbevölkerung ist die Sicherstellung der Ernährung, insbesondere die Versorgung mit Eiweiss ein grosses Problem (vgl. B. Andreae, Ernährungs-Umschau *29* (10) 324 bis 327 (1982)). Deshalb besteht die Aufgabe, tierische Eiweissproduktion mit einem Minimum an Futtermitteln zu bewirken, da hierbei z.B. Getreide der menschlichen Ernährung entzogen wird. Diese Aufgabe kann z.B. gelöst werden durch Wirkstoffe, die – in geringer Menge dem Futter beigemischt – das Wachstum, insbesondere die Gewichtszunahme, von Haus- und Nutztieren fördern oder die tierische Leistung, z.B. Eierlege- oder Milchleistung, positiv beeinflussen und damit den Verbrauch an Futtermitteln reduzieren.

Es sind eine Reihe von Stoffen in Gebrauch, die das Wachstum von Nutztieren fördern bzw. die Futterverwertung verbessern. Die wichtigsten Vertreter dieser Stoffe lassen sich beispielsweise aus dem Kapitel „Zusatzstoffe in Alleinfuttermitteln" aus dem Deutschen Futtermittelrecht entnehmen. Trotz der teils guten Wirkung dieser Verbindungen ist eine Reduktion der Aufwandmenge, insbesondere wegen des antibiotischen Charakters dieser Wirkstoffe wünschenswert.

Es wurde nun überraschenderweise gefunden, dass durch Kombination von Aminoreduktonen mit gebräuchlichen Wachstumsförderern eine Reduktion von deren Aufwandmenge möglich ist, und darüber hinaus eine wirkungsvolle Steigerung der Gewichtszunahme bzw. tierischen Leistung erzielt werden kann. Solche Aminoreduktone sind Verbindungen der allgemeinen Formel 1 oder deren physiologische unbedenklichen Salze, die in geringer Menge dem Futter bzw. den Tränkflüssigkeiten beigemischt werden

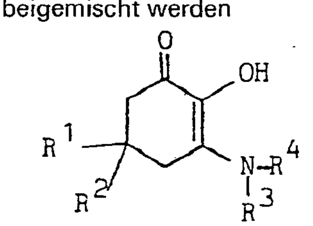

I

wobei

$R^1$, $R^2$ und $R^3$ gleich oder verschieden sind und Wasserstoff, Methyl oder Ethyl bedeuten und

$R^4$ eine geradkettige oder verzweigte $C_1$- bis $C_{20}$-Alkyl- oder Alkenylgruppe bedeutet, die eine oder mehrere Hydroxylgruppen, niedermolekulare Alkoxygruppen oder niedermolekulare Acyloxygruppen tragen kann, oder für einen Rest $-(CH_2-CH_2-O)_nH$ steht, in der n die Werte 1 bis 10 hat, der mit einem niedermolekularen Alkanol verethert oder mit einer niedermolekularen Carbonsäure verestert sein kann, oder den an die Aminogruppe gebundenen Rest einer natürlichen α- oder β-Aminosäure oder einen $C_1$- bis $C_{10}$-Alkylester davon bedeutet oder, falls es sich um eine α-Aminosäure handelt, um einen Rest, in welchem die Carboxylgruppe der Aminosäure und die 2-Hydroxylgruppe des Cyclohexenonrings ein Lacton bilden.

Als Wachstumsförderer (A) kommen z.B. Verbindungen der allgemeinen Formel II

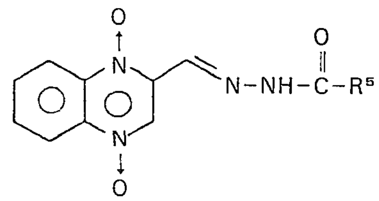

II

in Betracht, in der

$R^5$ einen $C_1$- bis $C_6$-Alkylrest, der durch Halogen oder Cyan substituiert sein kann, oder $C_1$- bis $C_6$-Alkoxy bedeutet.

Weiterhin sind Verbindungen der allgemeinen Formel III

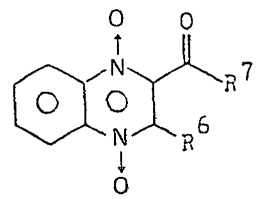

III

geeignet, in der

$R^6$ H; Wasserstoff oder einen niedermolekularen Alkylrest,

$R^7$ einen niedermolekularen Alkyl-, Alkoxy- oder Alkylaminrest bedeutet, wobei diese Reste gegebenenfalls noch Hydroxy- oder Alkoxygruppen tragen können.

Schliesslich sind insbesondere als Wachstumsförderer (A) Chinolinderivate der allgemeinen Formel IV a, b

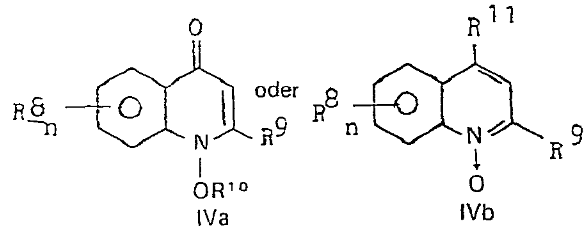

oder

IVa IVb

zu nennen, in denen

$R^8$ Wasserstoff, einen oder mehrere niedrigmolekulare Alkyl- oder Alkoxyreste oder Halogen,

$R^9$ Wasserstoff oder einen niedrigmolekularen Alkylrest und

$R^{10}$ und $R^{11}$ Wasserstoff, einen niedrigmolekularen aliphatischen oder einen araliphatischen Rest bedeuten.

Unter Reduktonen versteht man im allgemeineren Sinne stark reduzierende organische Substanzen, mit dem Strukturelement

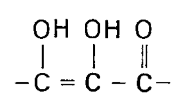

wobei die Hydroxylgruppen und der Sauerstoff der Oxogruppe auch durch die entsprechenden Aminfunktionen ersetzt werden können.

Verschiedentlich wurden Reduktone als Antioxidantien für oganische Materialien, insbesondere für Lebensmittel vorgeschlagen.

So untersuchten Obate et. al. (Nippon Nogei Kagaku Kaishi Band 45 (11), 1971, Seiten 489 bis 495; C.A. 77, 1972, Ref. Nr. 3944 m) Ascorbinsäure (L-3,4-Dihydroxy-5-(1,2-dihydroxyethyl)-2,5-dihydrofuran-2-on), Triosereduktоn (2,3-Dihydroxyacrolein), 2,3-Dihydroxy-pet-2-en-1-on und einige Aminosäurederivate des Triosereduktons

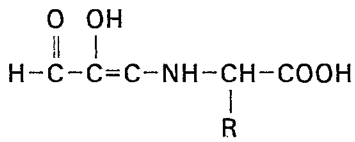

R=H; Methyl; Propyl-, Butyl-, Mercaptopropyl sowie in einer weiteren Arbeit (Nippon Nogei Kagaku Kaishi, Band 54 (7), 1980, Seiten 542 bis 544, C.A. 93, 1980, Ref. Nr. 166 211 q) verschiedene Anile des Triosereduktons.

Insgesamt ist beiden Arbeiten zu entnehmen, dass die Derivate des Triosereduktons als Zusatzstoffe für Lebens- und Futtermittel kaum in Betracht kommen, zumal auch die Herstellung dieser höchst empfindlichen Verbindungen grosse präparative Schwierigkeiten bereitet.

Aus der US-PS 3816137 sind ferner bestimmte 3-Aminoderivate des 2,3-Dihydroxy-cyclohex-2-en-1-ons als photographische Entwickler bekannt. Diese Aminoderivate leiten sich vom Methylamin, vom Dimethylamin sowie hauptsächlich von den heterocyclischen Aminen Piperidin und Morpholin ab. Die Herstellung dieser Reduktone erfolgt durch Umsetzung des betreffenden Amins mit 3-Chlor-cyclohexan-1,2-dion in Gegenwart von Triethylamin. Dieses Verfahren, welches von H. Simon et. al. näher beschrieben ist (Chem. Ber. 98, 1965, Seiten 3692 bis 3702), ist jedoch wegen der schlechten Zugänglichkeit der 3-Chlorcyclohexan-1,2-dione für technische Zwecke wenig geeignet und verläuft mit primären Aminen ausserdem nicht einheitlich.

Aus der Arbeit von Cocker et. al. (J. Chem. Soc. 1950, Seiten 2052 bis 2058) ist es weiterhin bekannt, Triosereduktоn mit aromatischen Aminen sowie mit Aminosäuren zu den entsprechenden 3-Substitutionsprodukten des 2,3-Dihydroxyacroleins umzusetzen. Im Falle der Aminosäuren sind die Ausbeuten mit etwa 10 bis 36%, bezogen auf das Triosereduktоn, jedoch unbefriedigend.

Demgegenüber erhält man die Aminoreduktone der Formel I nach der deutschen Patentanmeldung P 31 51 534.7 in einer bemerkenswert glatten Reaktion sowie in hohen Ausbeuten durch Umsetzung eines 2,3-Dihydroxy-cyclohex-2-en-1-ons der allgemeinen Formel V

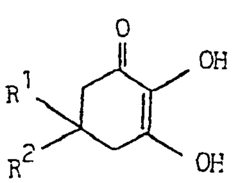

V

mit einer Aminoverbindung der allgemeinen Formel VI

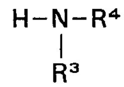

VI

oder einem mineralsauren Salz davon.

Die Ausgangsverbindungen der Formel V, unter denen diejenigen bevorzugt werden, in denen beide Reste $R^1$ und $R^2$ Wasserstoff bedeuten, sind bekannt oder nach bekannten Methoden erhältlich, z.B. nach dem in Houben-Weyl, Methoden der oganischen Chemie, Band 6/1 d, Seite 266 beschriebenen Verfahren durch Hydrierung von Pyrogallol in alkalischer Lösung.

Als Aminoverbindungen der Formel VI kommen vor allem diejenigen in Betracht, in denen $R^3$ Wasserstoff bedeutet und in denen sich $R^4$ von einer natürlichen α- oder β-Aminosäure mit mindestens einer primären Aminogruppe ableitet. Unter dem Begriff natürliche Aminosäuren sind, wie allgemein üblich, diejenigen zu verstehen, die in der Natur als Bausteine insbesondere von Proteinen oder als Stoffwechselprodukte vorkommen. Diese Säuren und auch deren $C_1$- bis $C_{10}$-Alkylester sind physiologisch unbedenklich und bedingen daher die physiologische Unbedenklichkeit der Verbindungen der Formel I, die beim Stoffwechsel zunächst zu Aminoverbindung der Formel VI und den nach bisherigen Feststellungen ebenfalls unbedenklichen Reduktonen der Formel V oder Oxidationsprodukten hiervon abgebaut werden können.

Die Umsetzungsprodukte der Verbindungen der Formel V mit den α-Aminosäuren sind entweder die entsprechenden freien Säuren der Formel VIIa oder deren Lactone der Formel VIIb

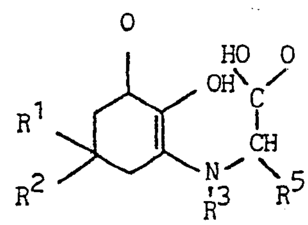

VIIa

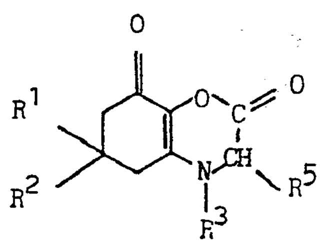

VIIb

wobei

$R^5$ den charakteristischen Rest der definitionsgemässen α-Aminosäuren bedeutet.

Eine weitere Gruppe von besonders geeigneten Aminoverbindungen der Formel V sind die $C_1$- bis $C_{20}$-Alkyl- und Alkenylamine, die auch Hydroxylgruppen tragen können, und deren N-Methyl- und N-Ethylderivate.

Als Verbindungen dieser Art kommen beispielsweise in Betracht:

Methylamin, Dimethylamin, Ethylamin, Ethylmethylamin, Diethylamin, Propylamin, Isopropylamin, Butylamin, Hexylamin, 2-Ethylhexylamin, Dodecylamin, Palmitylamin und Stearylamin.

Diese und die weiteren definitionsgemässen Amine können als Substituenten Hydroxylgruppen enthalten, die ihrerseits mittels eines $C_1$- bis $C_4$-Akanols oder einer $C_1$- bis $C_4$-Fettsäure verethert bzw. verestert sein können. Vorzugsweise befinden sich die Hydroxylgruppe bzw. die Alkoxygruppe oder die Acyloxygruppe in 2-Stellung zur Aminogruppe, da die entsprechenden Alkanolamine, beispielsweise Ethanolamin, Aminopropan-2-ol, N-Methylethanolamin, Aminobutan-2-ol, Aminohexan-2-ol und Aminododecan-2-ol leicht durch Umsetzung von den entsprechenden 1,2-Epoxyverbindungen, die ihrerseits aus den entsprechenden $\alpha$-Olefinen erhältlich sind, mit Ammoniak, Methylamin oder Ethylamin hergestellt werden können.

Zur Herstellung der Aminoreduktone der Formel I setzt man z.B. 1 mol einer Verbindung der Formel V mit 1 bis 3 mol, vorzugsweise mit äquimolaren oder annähernd äquimolaren Mengen der Amine der Formel VI entweder in Substanz oder in Gegenwart einer vorzugsweise aprotischen Flüssigkeit wie Toluol oder Xylol, in welcher die Reaktionspartner nicht gelöst zu sein brauchen, bei etwa 30 bis 150, besonders bei 50 bis 120° C miteinander um. Die Gegenwart von Wasser stört in einigen Fällen nicht und in den anderen wird das Wasser ohnehin zweckmässigerweise zusammen mit dem entstehenden Wasser im Laufe der Umsetzung entfernt.

Sofern eine $\alpha$-Aminosäure eingesetzt wird, erhält man entweder das offenkettige Derivat der Formel VIIa oder das zum Lacton cyclisierte Derivat der Formel VIIb. Ist Wasser zugegen, entsteht vorwiegend das offenkettige Derivat der Formel VIIa, und wird das Wasser entfernt, bildet sich meistens das Lacton der Formel VIIb. Häufig erhält man Mischungen von VIIa und VIIb, die in dieser Form verwendet werden können, da die Verbindungen in Gegenwart von Wasser – wie es ja unter physiologischen Bedingungen der Fall ist – zu der offenen Verbindung VIIa hydrolysieren.

Es empfiehlt sich, die Umsetzung durch Mitverwendung katalytischer Mengen einer Säure wie Chlorwasserstoff, p-Toluolsulfonsäure oder eines sauren Ionenaustauschers zu beschleunigen, jedoch gelingt die Reaktion auch ohne Säure.

Da es sich sowohl bei den Aminoreduktonen der Formel I als auch bei den Reduktonen der Formel V um oxidationsempfindliche Verbindungen handelt, versteht es sich von selbst, unter Ausschluss von Luftsauerstoff zu arbeiten, etwa indem man alle Operationen unter Stickstoff vornimmt.

Unter den Verbindungen der Formel I werden diejenigen bevorzugt, in denen $R^1$ und $R^2$ und $R^3$ Wasserstoff oder die Methylgruppe bedeuten. Hinsichtlich des Restes $R^4$ kommen besonders die einfachen Aminosäuren Glycin, Alanin, Valin, Leucin, Serin, Thyrosin und $\beta$-Alanin sowie die $C_2$- bis $C_{10}$-Alkylamine und deren 2-Hydroxyderivate und die Amine des Poylethertyps in Betracht.

Als Wachstumsförderer (A) kommen z.B. solche in Betracht, wie sie in dem obenerwähnten Kapitel „Zusatzstoffe in Alleinfuttermitteln" des Deutschen Futtermittelrechts aufgezählt werden. Besonders geeignet als Mischungspartner (A) sind die Wachstumsförderer aus der Klasse der Chinoxalin-di-N-oxide, die exemplarisch durch die Marktprodukte Carbadox, Olaquindox und Cyadox vertreten werden.

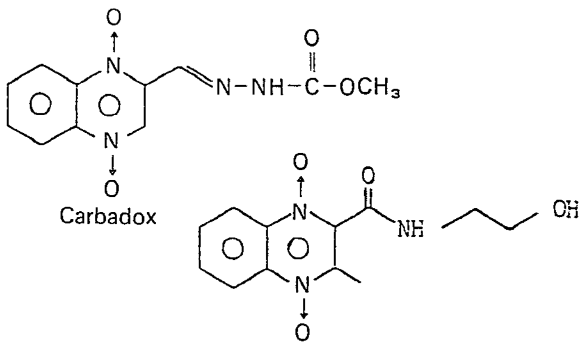

Carbadox

Olaquindox

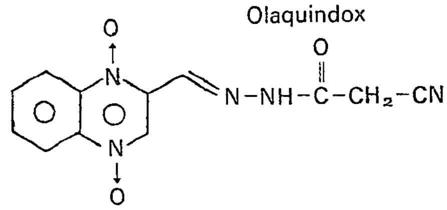

Cyadox

Besonders bevorzugt werden jedoch die in der deutschen Patentanmeldung P 31 19384.6 beschriebenen Chinolin-N-oxid-Derivate der allgemeinen Formeln IVa und IVb, da diese keine oder praktisch keine antibiotische Wirkung haben und deshalb die Mischung mit den nach bisherigen Kenntnissen untoxischen Aminoreduktonen der Formel I eine besonders umweltfreundliche Kombination ergibt.

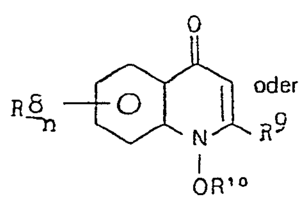

IVa

oder

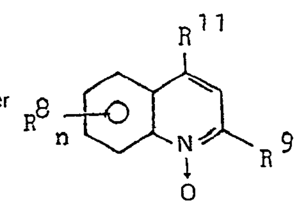

IVb

wobei $R^8$, $R^9$, $R^{10}$ und $R^{11}$ die eingangs angegebenen Bedeutungen haben.

Die Verbindungen der Formeln IVa und IVb können im Falle von $R^{10}$ bzw. $R^{11}$ = H in einem tautomeren Gleichgewicht vorliegen.

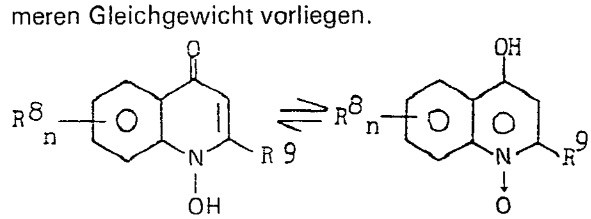

Die Verbindungen der Formeln IVa und IVb kann man beispielsweise aus den entsprechend

substituierten 4-Hydroxy-chinolinen der Formel VIII herstellen,

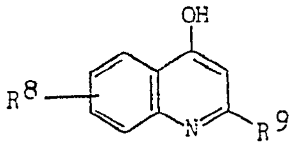

VIII

in der $R^8$ und $R^9$ die oben angegebenen Bedeutungen haben. Diese Hydroxychinoline erhält man in an sich bekannter Weise, z.B. durch Kondensation von entsprechenden Anilinen mit β-Ketoestern nach Gleichung (1) und (2), wie dies in der Deutschen Patentschrift 455387 beschrieben ist.

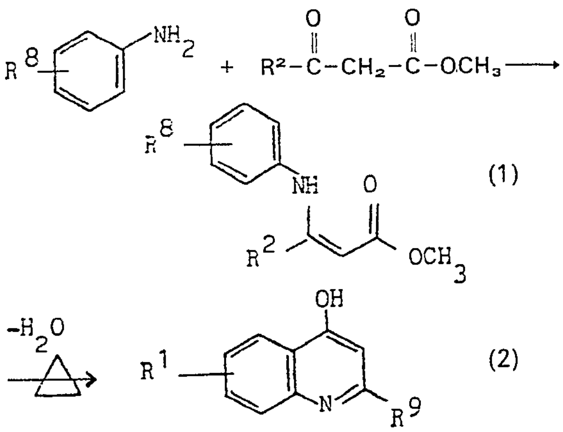

(1)

(2)

Die 4-Hydroxychinoline bzw. die durch Alkylierung erhaltenen 4-Alkoxy- und 4-Benzyloxychinoline können durch Persäuren oder $H_2O_2$ in essigsaurer Lösung, bevorzugt durch Peressigsäure, Perphthalsäure, m-Chlor-perbenzoesäure mit oder ohne Molybdän- oder Wolframsäure als Katalysator, zu den entsprechenden Verbindungen der Formeln IVa und IVb oxidiert werden. Im Falle der Oxidation der 4-Hydroxychinoline wird die 4-Hydroxygruppe zweckmässig durch Veresterung mit Essigsäure, Chlorkohlensäureester oder Benzoesäure geschützt. Nach erfolgter Oxidation kann diese Schutzgruppe leicht durch Rühren mit verdünnter Natron- oder Kalilauge bei Temperaturen von 0 bis 60° C, vorzugsweise 10 bis 30° C, entfernt werden. Durch anschliessendes Ansäuern wird das Produkt in guter Reinheit ausgefällt.

Die erfindungsgemässen Verbindungen der Formel IVa mit $R^{10} \neq H$ werden aus der Vorstufe ($R^{10} = H$) durch Alkylierung mit Dialkylsulfaten oder -sulfiten oder entsprechenden Alkylhalogeniden erhalten. Reaktionsmedium sind polare Lösungsmittel wie Tetrahydrofuran, Dioxan, Ether oder DMF, gegebenenfalls in Gegenwart von Basen und auch Wasser.

Die neue Wirkstoffkombination, bestehend aus Aminoredukton der Formel I und Wachstumsförderer (A), ist besonders bevorzugt, wenn die Komponente (A) eine Verbindung der Formeln II bis IV ist.

Die zur Erzielung des besten Wirkeffektes nötige Konzentration beträgt je Komponente 0,1 bis 100 ppm, besonders bevorzugt 0,1 bis 25 ppm, wobei das Verhältnis der Wirkstoffe (A) zu (B) 50 zu 1 bis 1 zu 50, vorzugsweise 10/1 bis 1/10 sein kann. Das optimale Verhältnis ist je nach Tierart verschieden und beträgt beispielsweise bei Ratten 1 : 1, wenn man beispielsweise eine Mischung der Verbindungen der Formeln

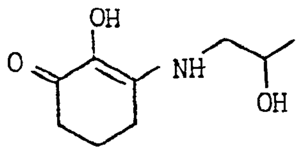

Aminoredukto

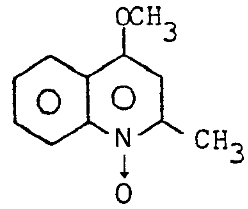

Chinolin-N-oxid

verwendet.

Die Wirkstoffkombination kann den Tieren mit dem üblichen Futter, aber auch mit dem Trinkwasser verabreicht werden. Zur Herstellung solcher Gemische eignen sich z.B. Vorgemische (bzw. Futterzusatzmittel) mit einem Gehalt von 1 bis 50% an Wirkstoffkombination. Obgleich natürlich die synergistischen Komponenten getrennt zu Vorgemischen verarbeitet und danach erst zum fertigen Futtermittel kombiniert werden können, erscheint es besonders vorteilhaft, die Wirkkombination bereits der Vormischung bzw. dem „Konzentrat" im richtigen Verhältnis zuzumischen und dann durch „Verdünnen" des Konzentrats durch das handelsübliche Futter die Endkonzentration einzustellen, da hierdurch die richtige Konzentration und das richtige Mischungsverhältnis im Endfutter gewährleistet wird. Die Futtervorgemische, Futtermittelkonzentrate und Futtermittel können als Träger jede Substanz pflanzlichen oder tierischen Ursprungs, die der Fütterung dient, enthalten. Zweckmässige Trägersubstanzen sind Weizengriess, Gerste, Roggen, Hafermehl, Reiskleie, Weizenkleie, Sojamehl, Maiskeimlingsmehl, Knochenmehl, Luzernenmehl, Sojagriess, Fleischmehl und Fischmehl sowie ihre Gemische.

Als Hilfsstoffe können die Futtervorgemische, Futtermittelkonzentrate und Futtermittel vorteilhaft Siliciumdioxid, Netzmittel, Antioxidationsmittel, Stärke, Dicalciumphosphat, Monocalciumphosphat, Calciumcarbonat und/oder Sorbinsäure enthalten. Die Netzmittel können z.B. nichttoxische Öle, vorteilhaft Soja-, Mais- oder Paraffinöl, sein. Als vorteilhafte Netzmittel haben sich auch die verschiedenen Alkylenglykole erwiesen. Die Stärke kann vorteilhaft Mais-, Weizen- oder Kartoffelstärke sein. Gegebenenfalls können die Verbindungen der Formeln I, II, III und IV auch auf Trägermaterialien aufgezogen, mit Gelatine umhüllt oder microverkapselt sein, und zwar einzeln, als auch in Kombination.

Durch Einmischen entsprechender Mengen der erfindungsgemässen Konzentrate in die für jede Tierart spezifische Futtermittelmischung kann jeweils die optimale Wirkstoffkombination und Wirkstoffmenge für jede Tierart eingestellt werden.

*Beispiele*

*Herstellung der Aminoreduktone*

Im folgenden wird der Rest 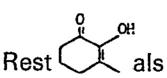 als Rest A be-

zeichnet. Alle Synthesen von Aminoreduktonen werden unter Stickstoff ausgeführt.

*Beispiel 1*

$A-NH-CH_2-COOH$

Eine Lösung aus 150 g (2 mol) Glycin und 450 ml Wasser wurde unter Rühren mit 106,5 g (0,83 mol) A-OH und 75 ml 2 n Salzsäure versetzt und zum Sieden erhitzt. Bei etwa 40° C entstand eine klare Lösung, aus der bei ungefähr 80° C das Verfahrensprodukt auszufallen begann. Nach Erreichen der Siedetemperature wurde das Gemisch auf 10° C abgekühlt und der Niederschlag, ein gelbliches Kristallpulver, wie üblich aufgearbeitet. Umkristallisation aus heissem Isopropanol lieferte das obengenannte Aminoredukton in 88%iger Ausbeute in Form farbloser Kristalle, Schmp. 185 bis 187° C.

*Beispiel 2*

$A-NH-CH_2-COO-C_2H_5$

Eine Mischung aus 128 g (1 mol) A-OH, 149,5 g (1 mol) Glycinethylesterhydrochlorid, 84 g (1 mol) $NaHCO_3$ und 1 l Toluol wurde etwa 2 Stunden unter laufender Auskreisung des Reaktionswassers zum Sieden erhitzt. Nach Abkühlung des Reaktionsgemisches auf 25° C wurde das entstandene NaCl abgetrennt und mit 1 l Ethanol gewaschen. Toluol- und Ethanolphase wurden vereinigt und unter vermindertem Druck bei 40° C eingeengt. Der Rückstand wurde aus 0,2 l Ethanol unter Zusatz von Aktivkohle umkristallisiert. Farblose Kristalle, Schmp. 121 bis 123° C; Ausbeute 60%.

*Beispiel 3*

$A-NH-n-C_{12}H_{25}$

Eine Mischung aus 145 g (0,78 mol) Dodecylamin, 100 g (0,78 mol) A-OH, 1 g p-Toluolsulfonsäure und 50 ml Toluol wurde etwa 1 Stunde unter Auskreisung des Reaktionswassers zum Sieden erhitzt und danach bei 60° C mit 500 ml n-Hexan versetzt. Beim weiteren Abkühlen schied sich das Verfahrensprodukt als gelblicher Kristallbrei ab. Die Ausbeute an der reinen, aus n-Hexan umkristallisierten Verbindung, die in Form farbloser Blättchen anfiel, betrug 81%; Schmp. 79 bis 81° C.

*Beispiele 4 bis 15*

$A-NR^3-R^4$

Analog Beispiel 3 wurden verschiedene Aminoverbindungen $HNR^3-R^4$ mit A-OH umgesetzt. Die Ergebnisse sind der Tabelle 1 zu entnehmen.

*Tabelle 1*

| Beispiel | Verbindung $A-NR^3-R^4$ | Schmp. °C | Ausbeute % |
|---|---|---|---|
| 4 | $A-NH-CH_2CH_2-OH$ | 164-165 | 63 |
| 5 | $A-NH-CH(CH_3)-COOH$ | 227-229 | 51 |
| 6 | $A-NH-CH_2-CH_2-COOH$ | 121-123 | 80 |
| 7 | $A-NH-CH_2-CH_2-COO-CH_3$ | 96-98 | 60 |
| 8 | $A-NH-CH_3$ | 175-178 | 48 |
| 9 | $A-NH-CH_2-CH(CH_3)-OH$ | 106-107 | 57 |
| 10 | $A-N(CH_3)-CH_2-CH_2-OH$ | flüssig | 64 |
| 11 | A-NH-n-hexyl | 101-102 | 70 |
| 12 | A-NH-n-tridecyl | 86-87 | 70 |
| 13 | A-NH-2-ethylhexyl | flüssig | 90 |
| 14 | A-N($CH_3$)-2-ethylhexyl | flüssig | 70 |
| 15 | $A-NH-(CH_2-CH_2-O)_2H$ | 85-89 | 52 |

*Beispiel 16*

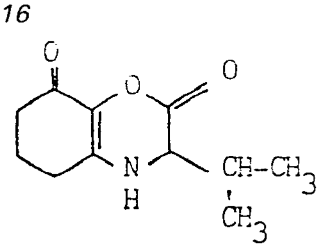

Eine Mischung aus 12,8 g (0,1 mol) A-OH, 12,9 g (0,11 mol) D,L-Valin, 50 ml Toluol und 1 g p-Toluolsulfonsäure wurde unter Auskreisen des Reaktionswassers etwa 2 Stunden lang zum Sieden erhitzt. In dieser Zeit wurden 3,6 ml (0,2 mol) Wasser abgeschieden.

Danach wurde das Reaktionsgemisch abgekühlt, wobei das Verfahrensprodukt auskristallisierte. Die Kristallmasse wurde abgetrennt und aus Ethanol umkristallisiert. Die Ausbeute an dem reinen Lacton betrug 57%; Schmp. 170 bis 171° C, farblose Kristalle.

*Herstellung von Wachstumsförderern*

*Beispiel 17*

86,5 g (0,5 Mol) 4-Methoxychinaldin (hergestellt aus 4-Hydroxychinaldin nach M. Marin, A. Ch. *4* [11], 301, 335 (1935)) werden bei Raumtemperatur in 250 ml Essigester gelöst und unter Rühren portionsweise mit 105 g (0,55 Mol) 90%iger 3-Chlorperbenzoesäure versetzt. Man erwärmt 4 Stunden auf 40°, rührt über Nacht bei Raumtemperatur nach, dabei fällt das Produkt aus. Nach Abkühlen im Eisbad wird abgesaugt, mit wenig kaltem Essigester gewaschen, der Filterkuchen dann in der gerade nötigen Menge Methanol gelöst und über einen basischen Ionenaustauscher (z.B. Lewatit MP 600, Aufwandmenge = 1 l) filtriert. Nach Einengen am Vakuum-Rotationsverdampfer erhält man 45,1 g leicht gelbliche Kristalle mit Fp. = 130 bis 131° C.

*Beispiel 18*

108,5 g (0,5 Mol) 6-Ethoxy-4-methoxychinaldin (hergestellt aus 6-Ethoxy-4-hydroxychinaldin nach M. Marin, A. Ch. *3* [11], 301, 335 (1935)) werden, wie in Beispiel 17 angegeben, in Essigester mit m-Chlorperbenzoesäure umgesetzt. Nach der Filtration über Lewatit MP 600 und Einengen am Vakuum-Rotationsverdampfer erhält man 79 g farblose Kristalle vom Fp. 134 bis 136° C.

*Beispiel 19*

80 g 4-Hydroxychinaldin (0,5 Mol) werden mit 27 g (0,5 Mol) $NaOCH_3$ in 400 ml trockenem Methanol in das Na-Salz überführt. Nach Entfernen des Methanols durch Abdampfen im Wasserstrahlvakuum wird das trockene Na-Salz in 700 ml trockenem Toluol suspendiert und bei 80° C langsam mit 54 g (0,5 Mol) Chlorameisensäureethylester versetzt. Nach 2 Stunden wird das Lösungsmittel im Wasserstrahlvakuum abdestilliert, der Rückstand 30 min in 600 ml Methylenchlorid aufgerührt und abgesaugt.

Das Filtrat versetzt man bei 15° C portionsweise mit 82,5 g (0,5 Mol) 85%iger m-Chlorperbenzoesäure. Nach einer Stunde wird mit kalter verdünnter Sodalösung und mit Wasser ausgeschüttelt, die organische Phase getrocknet und im Wasserstrahlvakuum eingeengt. Den Rückstand nimmt man in 200 ml Ethanol (50%ig) auf und versetzt bei Raumtemperatur mit einer Lösung von 16 g KOH in 200 ml Ethanol (50%ig). Nach 30 min wird auf das halbe Volumen eingeengt, das Produkt durch Ansäuern mit 2 n HCl auf pH 4 ausgefällt. Man erhält 47 g bis 60 g Rohprodukt. Dies kann gegebenenfalls aus Methanol umkristallisiert werden zum reinen 4-Hydroxychinaldin-N-oxid. Fp.: 248 bis 250° C.

*Beispiel 20*

14 g (0,08 Mol) 4-Hydroxychinaldin-N-oxid werden in 400 ml Dioxan suspendiert und mit 4,8 g (0,12 Mol) NaOH in 80 ml $H_2O$ versetzt. Man rührt bei 50° C und tropft 13,2 g (0,116 Mol) Dimethylsulfat in 80 ml Dioxan zu. Nach 2 Stunden wird die Lösung im Wasserstrahlvakuum eingeengt, der Rückstand in 20 ml Wasser aufgenommen und mit conc. HCl auf pH 1 angesäuert. Nach Absaugen und Trocknen erhält man 14 g Rohprodukt, das aus Wasser unter Zusatz von Aktiv-Kohle umkristallisiert werden kann.

Ausbeute: 6 bis 8 g N-Methoxy-chinaldon-4, Fp.: 186 bis 188° C/Zersetzung.

*Beispiele 21 bis 28*

In Analogie zu den Angaben der Beispiele 17 bis 20 werden die folgenden Verbindungen erhalten:

| | | Fp. [° C] | Analyse |
|---|---|---|---|
| 21 | 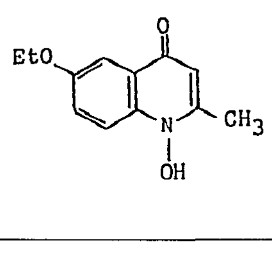 | 248-250 | Berechnet: C 65,74 H 5,98 N 6,39<br>Gefunden: C 65,7 H 6,0 N 6,4 |
| 22 | 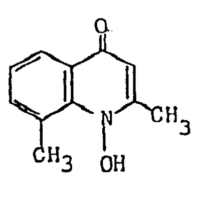 | 253-254 | Berechnet: C 69,83 H 5,86 N 7,40<br>Gefunden: C 70,0 H 6,1 N 7,7 |

| | | Fp. [°C] | Analyse |
|---|---|---|---|
| 23 | 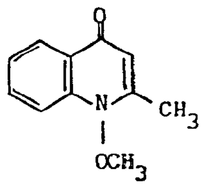 | 186-188 (Zers.) | Berechnet: C 69,84 H 5,86 N 7,40<br>Gefunden: C 70,1 H 6,0 N 7,5 |
| 24 | 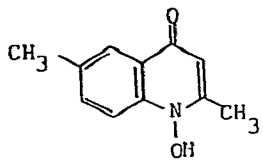 | 248-252 | Berechnet: C 69,83 H 5,86 N 7,40<br>Gefunden: C 70,3 H 5,9 N 7,6 |
| 25 | 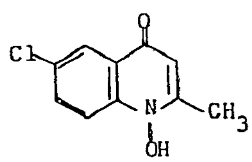 | 265-267 (Zers.) | Berechnet: C 57,28 H 3,82 N 6,68<br>Gefunden: C 57,1 H 3,8 N 6,7 |
| 26 | 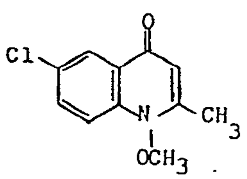 | 128-133 | Berechnet: C 59,06 H 4,47 N 6,26<br>Gefunden: C 59,3 H 4,7 N 6,3 |
| 27 | 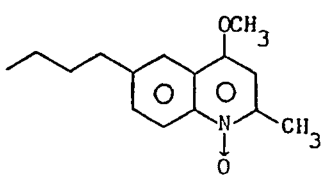 | 1/4 $H_2O$ 65-68 | Berechnet: C 72,1 H 7,81 N 5,61<br>Gefunden: C 71,8 H 8,0 N 5,6 |
| 28 | 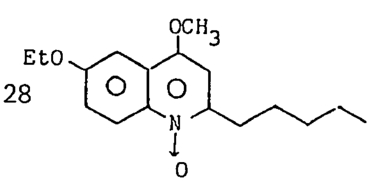 | 106 | Berechnet: C 68,4 H 8,55 N 5,2<br>Gefunden: C 68,3 H 8,7 N 5,1 |

*Anwendungsbeispiele*

*Beispiel 29*

Für Ferkel wurde eine Vormischung (Premix) der folgenden Zusammensetzung bereitet:

| Bestandteile | Menge |
|---|---|
| Vitamin A | 1 200000 IE |
| Vitamin $D_3$ | 300000 IE |
| Vitamin E | 2000 IE |
| Vitamin $B_2$ | 600 mg |
| Vitamin $B_3$ | 2000 mg |
| Vitamin $B_{12}$ | 5 mg |
| Nicotinsäure (Niacin) | 3000 mg |
| Cholinchlorid | 40000 mg |
| 4-Methoxy-chinaldin-N-oxid (aus Beispiel 17) | 1 000 mg |
| 3-Isopropanolamino-2-hydroxy-cyclohexan (aus Beispiel 9) | 1 000 mg |
| Butylhydroxytoluol (Antioxidationsmittel) | 30000 mg |
| Spurenelemente | |
| Mangan | 6000 mg |
| Eisen | 10000 mg |
| Zink | 15000 mg |
| Kupfer | 30000 mg |
| Iod | 100 mg |
| zweimal gemahlene Kleie zum Auffüllen auf | 1 000 g |

Diese Vitamin- und Spurenelementenvormischung wurde dem Basisfuttermittel in einer Menge von 0,5 kg je 100 kg zugesetzt.

*Beispiel 30*

0,5 kg der nach dem Beispiel 29 bereiteten Vormischung wurden einem Grundfuttermittel der folgenden Zusammensetzung zugemischt:

| Bestandteil | Menge |
|---|---|
| Mais | 25,0 kg |
| Weizen | 34,0 kg |
| Extrahierte Soja | 18,0 kg |
| Milchpulver | 9,9 kg |
| Fischmehl | 4,0 kg |
| Futterhefe | 2,0 kg |
| Fettpulver | 3,4 kg |
| Mineralvormischung (Dicalciumphosphat/Monocalciumphosphat/Calciumcarbonat = 55/40/5) | 1,8 kg |
| Futterkalk | 1,0 kg |
| Natriumchlorid von Futterqualität | 0,4 kg |
| Vormischung des Beispiels 29 | 0,5 kg |
| Gesamtgewicht | 100,0 kg |

Der Wirkstoffgehalt des so erhaltenen Ferkelfuttermittels betrug je 5 ppm = 0,0005 Gew.% an jeder synergistischen Komponente (aus Beispiel 9 und 17).

*Beispiel 31*

Die Wirksamkeit der erfindungsgemässen Futtermittel hinsichtlich einer Verbesserung der Wachstumsleistung sei durch einen Fütterungsversuch an Ratten illustriert:

Die Versuchstiere, männliche SPF-Wistar-Ratten (Firma Hagemann, Extertal) wurden jeweils zu dritt in Makrolonkäfigen gehalten und mit handelsüblichem Versuchsfutter (Eggersmann, Rinteln) gefüttert. Futter und Wasser standen ohne Mengenbegrenzung zur Verfügung.

Die Ergebnisse enthält Tabelle 2.

*Beispiel 32*

Ein Fütterungsversuch an Schweinen zeigt die Wirkungssteigerung der erfindungsgemässen Stoffmischung im Vergleich zu handelsüblichen Wirkstoffen (Tabelle 3):

*Tabelle 2*

Fütterungsversuche an Ratten (SPF-Wistar):

| Wirkstoffkombination A x) | Wirkstoffkombination B x) | Konzentration im Futter A | Konzentration im Futter B | Tierzahl n | Lebendgewicht in g 0. Tag | Lebendgewicht in g 21. Tag | Veränderung gegenüber Kontrolle = 100% |
|---|---|---|---|---|---|---|---|
| Kontrolle | | — | — | 250 | 62,7±1,5 | 178,0± 3,0 | ±0 |
| Carbadox | — | 50 ppm | — | 20 | 62 ±2,3 | 184,1± 6,0 | + 5,5% |
| Carbadox | — | 25 ppm | — | 40 | 62,5±2,3 | 177,7± 2,4 | + 0,1% |
| Carbadox | 9 | 50 ppm | 20 ppm | 9 | 63,2±2,9 | 184,6± 8,9 | + 5,3% |
| Carbadox | 9 | 25 ppm | 20 ppm | 10 | 62,9±2,2 | 186,4± 9,2 | + 7,1% |
| Carbadox | 9 | 10 ppm | 10 ppm | 10 | 62,6±3,4 | 187,4± 8 | + 8,2% |
| 21 | — | 50 ppm | — | 10 | 63,3±3,6 | 178,4±11,3 | ± 0% |
| 21 | 9 | 50 ppm | 10 ppm | 10 | 62,1±2,5 | 182,2± 9,9 | + 4,2% |
| 17 | — | 25 ppm | — | 10 | 65,0±5 | 180,9± 6,2 | + 0,5% |
| 17 | — | 10 ppm | — | 10 | 62,9±2,1 | 180,6± 8,8 | + 2,1% |
| 17 | 9 | 10 ppm | 10 ppm | 20 | 62,6±2,2 | 187,2±13 | + 8,1% |
| 18 | 9 | 50 ppm | 20 ppm | 10 | 63,2±3,8 | 185,0±11 | + 5,6% |
| 18 | 9 | 5 ppm | 10 ppm | 10 | 64,3±4,3 | 191,2±12 | +10 % |
| 17 | 6 | 10 ppm | 10 ppm | 10 | 62,7±2,3 | 186,3±11 | + 7,2% |
| 17 | 3 | 10 ppm | 10 ppm | 10 | 62,1±3,8 | 185,4± 9 | + 6,9% |

x) Nr. des Synthese-Beispiels

*Tabelle 3*

| Wirkstoffkombination A x) | B x) | Konzentration im Futter A | B | Tierzahl | Anfangs-Lebensgewicht kg | nach 58 Tagen kg | Veränderung gegenüber Kontrolle |
|---|---|---|---|---|---|---|---|
| Kontrolle | | — | — | 5♂ 5♀ | 14,5±0,7 | 42,9± 5,8 | ± 0 % |
| Carbadox | — | 25 ppm | | 5♂ 5♀ | 14,4±0,9 | 45,7± 7,4 | +10,2% |
| 17 | — | 6 ppm | | 4♂ 4♀ | 14,4±0,6 | 42,3± 6,3 | − 1,7% |
| 17 | 9 | 6 ppm | 12 ppm | 5♂ 5♀ | 14,5±1,1 | 46,2± 5,5 | +11,6% |
| Kontrolle | — | — | — | 5♂ 5♀ | 12,5±1,3 | 41,9± 5,8 | ± 0 % |
| 18 | — | 6 ppm | — | 5♂ 5♀ | 12,1±2,0 | 43,5±10,5 | + 6,8% |
| 18 | 9 | 6 ppm | 8 ppm | 5♂ 5♀ | 12,1±0,2 | 45,3± 5,5 | +12,9% |
| Carbadox | — | 25 ppm | — | 5♂ 5♀ | 13,7±1,0 | 45,0± 7,7 | + 6,4% |
| Carbadox | 9 | 10 ppm | 8 ppm | 5♂ 5♀ | 13,9±0,9 | 45,4± 6,5 | + 7,2% |
| Kontrolle | — | — | — | 9♂ | 13,5±1,3 | 64,5± 8,0 | ± 0 % |
| Carbadox | — | 25 ppm | — | 4♂ | 14,4±0,9 | 73,8±11,3 | +14,4% |
| 17 | 9 | 6 ppm | 12 ppm | 5♂ | 14,5±1,0 | 83,7± 7,5 | +29,7% |
| 17 | 9 | 6 ppm | 8 ppm | 5♂ | 12,1±0,3 | 81,0± 9,0 | +25,6% |

x) Nr. des Synthese-Beispiels

**Patentansprüche**

1. Wachstumsfördernde Stoffmischung, enthaltend

(A) einen handelsüblichen Wachstumsförderer sowie

(B) ein Aminoredukton der allgemeinen Formel I

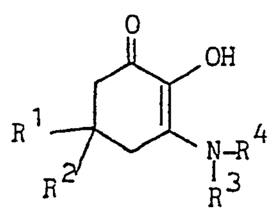

I

oder eines physiologisch verträglichen mineralsauren Salzes davon, wobei

$R^1$, $R^2$ und $R^3$ gleich oder verschieden sind und Wasserstoff, Methyl oder Ethyl bedeuten und

$R^4$ eine geradkettige oder verzweigte $C_1$- bis $C_{20}$-Alkyl- oder Alkenylgruppe bedeutet, die eine oder mehrere Hydroxylgruppen, niedermolekulare Alkoxygruppen oder niedermolekulare Acyloxygruppen tragen kann, oder für einen Rest $-(CH_2-CH_2-O)_nH$ steht, in dem n die Werte 1 bis 10 hat, der mit einem niedermolekularen Alkanol verethert oder mit einer niedermolekularen Carbonsäure verestert sein kann, oder den an die Aminogruppe gebundenen Rest einer natürlichen α- oder β-Aminosäure oder einen $C_1$- bis $C_{10}$-Alkylester davon oder, falls es sich um eine α-Aminosäure handelt, um einen Rest, in welchem die Carboxylgruppe der Aminosäure und die 2-Hydroxylgruppe des Cyclohexenonrings ein Lacton bilden, bedeutet.

2. Stoffmischung gemäss Anspruch 1, dadurch gekennzeichnet, dass das Gewichtsverhältnis zwischen Wachstumsförderer (A) und Aminoredukton (B) 50:1 bis 1:50 beträgt.

3. Stoffmischung gemäss Anspruch 1, enthaltend als Wachstumsförderer (A) Verbindungen der allgemeinen Formel II

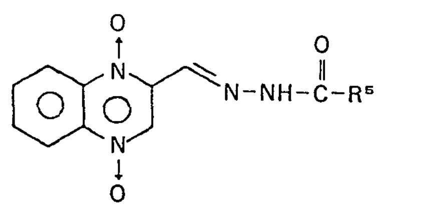

II

in der

$R^5$ einen $C_1$- bis $C_6$-Alkylrest, der durch Halogen oder Cyan substituiert sein kann, oder $C_1$- bis $C_6$-Alkoxy bedeutet.

4. Stoffmischung gemäss Anspruch 1, enthaltend als Wachstumsförderer (A) Verbindungen der allgemeinen Formel III

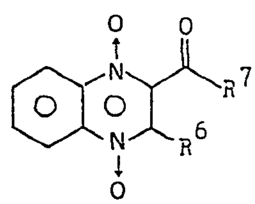

III

in der

$R^6$ Wasserstoff oder einen niedermolekularen Alkylrest,

$R^7$ einen niedermolekularen Alkyl-, Alkoxy- oder Alkylaminrest bedeutet, wobei diese Reste gegebenenfalls noch Hydroxy- oder Alkoxygruppen tragen können.

5. Stoffmischung gemäss Anspruch 1, enthaltend als Wachstumsförderer (A) ein Chinolinderivat der allgemeinen Formel IV a, b

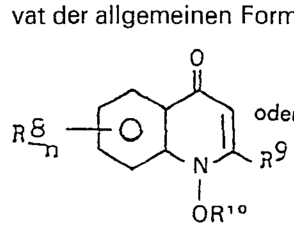

oder

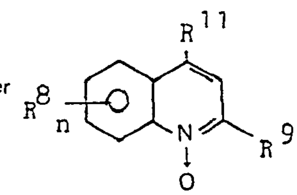

IVa IVb

in denen

$R^8$ Wasserstoff, einen oder mehrere niedrigmolekulare Alkyl- oder Alkoxyreste oder Halogen,

$R^9$ Wasserstoff oder einen niedrigmolekularen Alkylrest und

$R^{10}$ und $R^{11}$ Wasserstoff, einen niedrigmolekularen aliphatischen oder einen araliphatischen Rest bedeuten.

6. Stoffmischung bestehend aus

(A) einer oder mehrerer der Verbindungen 4-Hydroxy-chinaldin-N-oxid, 6-Methyl-4-hydroxy-chinaldin-N-oxid, 6-Ethoxy-4-hydroxychinaldin-N-oxid, 6-Ethoxy-4-methoxychinaldin-N-oxid, 6-Chlor-4-hydroxy-chinaldin-N-oxid, 8-Methyl-4-hydroxychinaldin-N-oxid, 6-Butyl-4-Methoxy-chinaldin-N-oxid, 6-Ethoxy-4-methoxy-2-pentyl-chinolin-N-oxid und

(B) einer oder mehrerer der Verbindungen 3-Ethanolamino-2-hydroxy-cyclohexenon, 3-Isopropanolamino-2-hydroxy-cyclohexenon, 3-β-Alanino-2-hydroxy-cyclohexenon, 3-Glycino-2-hydroxy-cyclohexenon, 3-Dodecylamino-2-hydroxy-cyclohexenon, 3-(β-Hydroxy-dodecylamino)-2-hydroxy-cyclohexenon, wobei das Verhältnis (A) zu (B) 50:1 bis 1:50 beträgt.

7. Stoffmischung gemäss Anspruch 1 in Form eines Trockenpulvers, dadurch gekennzeichnet, dass die Bestandteile (A) und (B) in gecoateter oder microverkapselter Form, gegebenenfalls in Mischung mit Trägermaterialien, Antioxidantien, Farb- und Geschmackstoffen vorliegen.

8. Wachstumsförderndes Futtermittel oder Tränkflüssigkeit, enthaltend, neben der Hauptmenge eines üblichen Futtermittelvorgemisches, Futtermittelkonzentrates oder Futtermittels bzw. Wasser, geringe Mengen einer Stoffmischung gemäss Ansprüchen 1 bis 7.

9. Futtermittel oder Tränkflüssigkeit gemäss Anspruch 8, enthaltend 0,1 bis 500 ppm einer Stoffmischung nach Ansprüchen 1 bis 7.

10. Futtermittel oder Tränkflüssigkeit gemäss Anspruch 8, enthaltend 0,1 bis 500 ppm einer Verbindung der allgemeinen Formel I und 0,1 bis 500 ppm einer Verbindung der allgemeinen Formel II.

11. Futtermittel oder Tränkflüssigkeit gemäss Anspruch 8, enthaltend 0,1 bis 500 ppm einer Verbindung der allgemeinen Formel I und 0,1 bis 500 ppm einer Verbindung der allgemeinen Formel III.

12. Futtermittel oder Tränkflüssigkeit gemäss Anspruch 8, enthaltend 0,1 bis 500 ppm einer Verbindung der allgemeinen Formel I und 0,1 bis 500 ppm einer Verbindung der allgemeinen Formel IVa, b.

## Claims

1. A growth-promoting mixture which contains

(A) a commercial growth promoter, and

(B) an aminoreductone of the general formula I

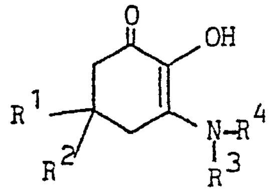

I

where

$R^1$, $R^2$ and $R^3$ are identical or different and are each hydrogen, methyl or ethyl, and

$R^4$ is a straight-chain or branched $C_1$-$C_{20}$-alkyl or alkenyl group which can carry one or more hydroxyl groups, low molecular weight alkoxy groups or low molecular weight acyloxy groups, or is a radical $-(CH_2-CH_2-O)_nH$, where n is from 1 to 10, which can be etherified with a low molecular weight alkanol or esterified with a low molecular weight carboxylic acid, or is the radical, bonded at the amino group, of a natural α- or β-aminoacid or a $C_1$-$C_{10}$-alkyl ester thereof, or, in the case of an α-aminoacid, is a radical in which the carboxyl group of the aminoacid and the 2-hydroxyl group of the cyclohexenone ring form a lactone, or its physiologically tolerated salt with a mineral acid.

2. A mixture as claimed in claim 1, wherein the weight ratio of the growth promoter (A) to the aminoreductone (B) is from 50:1 to 1:50.

3. A mixture as claimed in claim 1, which contains, as the growth promoter (A), a compound of the general formula II

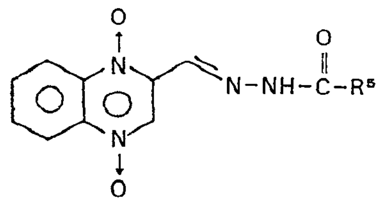

II

where

$R^5$ is $C_1$-$C_6$-alkyl which can be substituted by halogen or cyano, or is $C_1$-$C_6$-alkoxy.

4. A mixture as claimed in claim 1, which contains, as the growth promoter (A), a compound of the general formula III

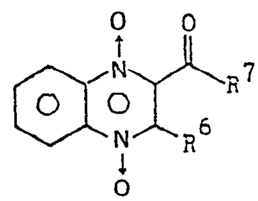

III

where

$R^6$ is hydrogen or a low molecular weight alkyl radical, and

$R^7$ is a low molecular weight alkyl, alkoxy or alkylamine radical, these radicals being unsubstituted or substituted by hydroxyl or alkoxy groups.

5. A mixture as claimed in claim 1, which contains, as the growth promoter (A), a quinoline derivative of the general formula IV a or b

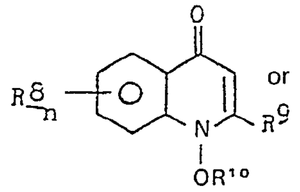

or

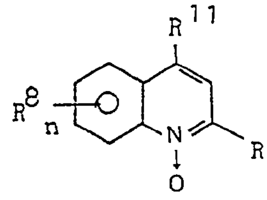

IVa IVb

where

$R^8$ is hydrogen, one or more low molecular weight alkyl or alkoxy radicals or halogen,

$R^9$ is hydrogen or a low molecular weight alkyl radical, and

$R^{10}$ and $R^{11}$ are each hydrogen, a low molecular weight aliphatic radical or an araliphatic radical.

6. A mixture which consists of

(A) one or more of the compounds 4-hydroxyquinaldine N-oxide, 6-methyl-4-hydroxyquinaldine N-oxide, 6-ethoxy-4-hydroxyquinaldine N-oxide, 6-ethoxy-4-methoxyquinaldine N-oxide, 6-chloro-4-hydroxyquinaldine N-oxide, 8-methyl-4-hydroxyquinaldine N-oxide, 6-butyl-4-methoxyquinaldine N-oxide and 6-ethoxy-4-methoxy-2-pentylquinoline N-oxide, and

(B) one or more of the compounds 3-ethanolamino-2-hydroxy-cyclohexenone, 3-isopropanolamino-2-hydroxycyclohexenone, 3-β-alanino-2-hydroxycyclohexenone, 3-glycino-2-hydroxycyclohexenone, 3-dodecylamino-2-hydroxycyclohexenone and 3-(β-hydroxydodecylamino)-2-hydroxycyclohexenone, the ratio of (A) to (B) being from 50:1 to 1:50.

7. A mixture as claimed in claim 1, in the form of a dry powder, wherein the components (A) and (B) are present in coated or microencapsulated form, optionally in admixture with carriers, antioxidants, colorants and flavoring agents.

8. A growth-promoting feed or drinking liquid which contains, in addition to a conventional feed premix, feed concentrate or feed, or water, which is present as the principal component, a small amount of a mixture as claimed in claims 1 to 7.

9. A feed or drinking liquid as claimed in claim 8, which contains from 0.1 to 500 ppm of a mixture as claimed in claims 1 to 7.

10. A feed or drinking liquid as claimed in claim 8, which contains from 0.1 to 500 ppm of a compound of the general formula I and from 0.1 to 500 ppm of a compound of the general formula II.

11. A feed or drinking liquid as claimed in claim 8, which contains from 0.1 to 500 ppm of a compound of the general formula I and from 0.1 to 500 ppm of a compound of the general formula III.

12. A feed or drinking liquid as claimed in claim 8, which contains from 0.1 to 500 ppm of a compound of the general formula I and from 0.1 to 500 ppm of a compound of the general formula IV a or b.

## Revendications

1. Mélange favorable à la croissance, renfermant

(A) un produit commercial favorsiant la croissance, ainsi que

(B) une «réductone» aminée de formule générale I:

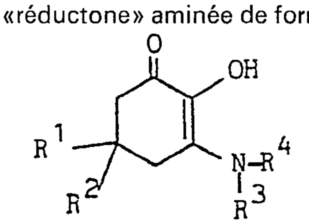

I

ou d'un sel d'acide minéral de celle-ci, physiologiquement compatible, et dans lequel:

$R^1$, $R^2$ et $R^3$ sont identiques ou différents et désignent l'hydrogène ou un groupe méthyle ou éthyle, et

$R^4$ représente un groupe alkyle ou alcényle à chaîne droite ou ramifiée en $C_1$ à $C_{20}$, qui peut porter un ou plusieurs groupes hydroxy, groupes alcoxy à faible poids moléculaire ou groupes acyloxy à faible poids moléculaire, ou est mis pour un reste $-(CH_2-CH_2-O)_nH$, dans lequel n a une valeur de 1 à 10, qui peut être étherifié par un alcanol à faible poids moléculaire ou estérifié par un alcanol à faible poid moléculaire ou estérifié par un acide carboxylique à faible poids moléculaire, ou bien désigne le reste, combiné au groupe amino, d'un α- ou β-amino-acide naturel ou un ester alkylique en $C_1$ à $C_{10}$ de celui-ci, ou bien, dans le cas où il s'agit d'un α-amino-acide, désigne un reste dans lequel le groupe carboxyle de l'amino-acide et le groupe α-hydroxyle du noyau cyclohexénone forment une lactone.

2. Mélange de substances selon la revendication 1, caractérisé par le fait que le rapport pondéral entre le produit favorisant la croissance (A) et la «réductone» aminée (B) va de 50:1 à 1:50.

3. Mélange de substances selon la revendication 1 renfermant, comme produit favorisant la croissance (A), des composés de formule générale II

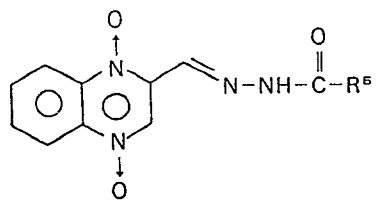

II

dans laquelle $R^5$ représente un reste alkyle en $C_1$ à $C_6$ qui peut être substitué par un radical halogène ou cyano, ou représente un groupe alcoxy en $C_1$ à $C_6$.

4. Mélange de substances selon la revendication 1, renfermant comme produit favororisant la croissance (A) des composés de la formule générale III

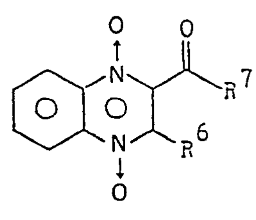

III

dans laquelle

$R^6$ représente de l'hydrogène ou un reste alkyle à faible poids moléculaire,

$R^7$ représente un reste alkyle, alcoxy ou alkylamino à faible poids moléculaire, ces restes pouvant porter encore, le cas échéant, des groupes hydroxy ou alcoxy.

5. Mélange de substances selon la revendication 1, renfermant comme produit favorisant la croissance (A) un dérivé de quinoléine de formule générale IV a, b

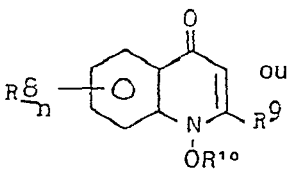
ou
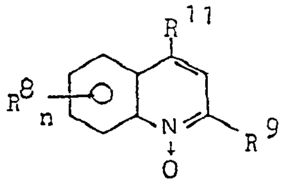

IVa IVb

dans lesquelles

$R^8$ désigne l'hydrogène, un ou plusieurs restes alkyle ou alcoxy à faible poids moléculaire, ou un halogène,

$R^9$ désigne l'hydrogène ou un reste alkyle à faible poids moléculaire,

$R^{10}$ et $R^{11}$ désignent l'hydrogène, un reste aliphatique à faible poids moléculaire ou un reste araliphatique.

6. Mélange de substances consistant en

(A) un ou plusieurs des composés N-oxyde de 4-hydroxyquinaldine, N-oxyde de 6-méthyl-4-hydroxyquinaldine, N-oxyde de 6-éthoxy-4-hydroxyquinaldine, N-oxyde de 6-éthoxy-4-méthoxyquinaldine, N-oxyde de 6-chloro-4-hydroxyquinaldine, N-oxyde de 8-méthyl-4-hydroxyquinaldine, N-oxyde de 6-butyl-4-méthoxyquinaldine, N-oxyde de 6-éthoxy-4-méthoxy-2-pentyl-quinoléine, et

(B) un ou plusieurs des composés 3-éthanolamino-2-hydroxy-cyclohexénone, 3-isopropanolamino-2-hydroxy-cyclohexénone, 3-β-alanino-2-hydroxy-cyclohexénone, 3-glycino-2-hydroxy-cyclohexénone, 3-dodécylamino-2-hydroxy-cyclohexénone, 3-(β-hydroxydodécylamino)-2-hydroxy-cyclohexénone, le rapport (A):(B) étant de 50:1 à 1:50.

7. Mélange de substances selon la revendication 1 sous forme de poudre sèche, caractérisé par le fait que les composants (A) et (B) sont présents sous une forme enrobée ou microencapsulée, éventuellement mélangés à des supports, des antioxygènes, des matières colorantes et des arômes artificiels.

8. Produit de fourrage ou liquide d'abreuvage favorable à la croissance, renfermant, outre la quantité principale d'un prémélange usuel de produit de fourrage, d'un concentré de produit de fourrage ou d'un produit de fourrage, ou encore d'eau, de faibles quantités d'un mélange de substances selon les revendications 1 à 7.

9. Produit de fourrage ou liquide d'abreuvage selon la revendication 8, renfermant de 0,1 à 500 ppm d'un mélange de substances selon les revendications 1 à 7.

10. Produit de fourrage ou liquide d'abreuvage selon la revendication 8, renfermant de 0,1 à 500 ppm d'un composé de formule générale I et de 0,1 à 500 ppm d'un composé de formule générale II.

11. Produit de fourrage ou liquide d'abreuvage selon la revendication 8, renfermant de 0,1 à 500 ppm d'un composé de formule générale I et de 0,1 à 500 ppm d'un composé de formule générale III.

12. Produit de fourrage ou liquide d'abreuvage selon la revendication 8, renfermant de 0,1 à 500 ppm d'un composé de formule générale I et de 0,1 à 500 ppm d'un composé de formule générale IV a, b.